Europäisches Patentamt

European Patent Office

Office européen des brevets

(11) Veröffentlichungsnummer: **0 038 793**
**B1**

## (12) EUROPÄISCHE PATENTSCHRIFT

(45) Veröffentlichungstag der Patentschrift:
**23.11.83**

(21) Anmeldenummer: **81890061.5**

(22) Anmeldetag: **08.04.81**

(51) Int. Cl.³: **A 41 D 31/00**, A 44 C 5/00,
A 61 F 13/00

(54) Gegenstand aus insbesondere flexiblem Werkstoff.

(30) Priorität: **18.04.80 AT 2101/80**
**18.04.80 AT 2102/80**
**03.11.80 AT 5400/80**

(43) Veröffentlichungstag der Anmeldung:
**28.10.81 Patentblatt 81/43**

(45) Bekanntmachung des Hinweises auf die Patenterteilung:
**23.11.83 Patentblatt 83/47**

(84) Benannte Vertragsstaaten:
**BE CH DE GB LI SE**

(56) Entgegenhaltungen:
**BE - A - 516 756**
**DE - A - 2 209 281**
**DE - B - 1 173 615**
**FR - A - 1 133 472**
**FR - A - 1 370 184**
**FR - A - 2 268 540**
**GB - A - 446 943**

(73) Patentinhaber: **Hermann Hirsch Leder- und Kunststoffwarenfabrik, Lastenstrasse 34,
A-9021 Klagenfurt (Kärnten) (AT)**

(72) Erfinder: **Hirsch, Hermann, Lastenstrasse 34,
A-9021 Klagenfurt (AT)**
Erfinder: **Jarisch, Reinhart, Dr., Alserstrasse 4,
A-1090 Wien (AT)**

(74) Vertreter: **Beer, Otto, Dipl.-Ing. et al, Lindengasse 8,
A-1071 Wien (AT)**

Gegenstand aus insbesondere flexiblem Werkstoff

Die Erfindung betrifft einen Gegenstand aus insbesondere flexiblem Werkstoff, der bei seiner bestimmungsgemässen Verwendung auf der Haut oder der Schleimhaut eines Trägers unmittelbar aufliegt und zumindest in seinem bei bestimmungsgemässer Verwendung auf der Haut oder der Schleimhaut des Trägers aufliegenden Bereich eine Schichte trägt, die aus Metall, insbesondere Aluminium oder einem Edelmetall, vorzugsweise Silber, oder aus Metallpartikeln, insbesondere Aluminium oder einem Edelmetall, vorzugsweise Silber, besteht, die in ein Bindemittel eingebettet sind.

Es ist bekannt, dass bestimmte Werkstoffe Allergene und/oder Irritantien enthalten, die beim Tragen von aus solchen Werkstoffen gefertigten Gegenständen bei zu Hautreizungen neigenden Personen zu unangenehmen Hautreizungen und/oder Allergien führen können. Zu diesen Werkstoffen zählen unter anderen Leder und lederähnliche Werkstoffe, aus welchen z.B. Handschuhe, Schweissbänder für Hüte oder Stahlhelme und Uhrbänder sowie orthopädische Waren hergestellt werden. Ähnliches gilt auch für Kunststoffe z.B. in Folien- oder Gewebeform sowie für Gummi und gummiähnliche Werkstoffe. Zu diesen Werkstoffe zählen auch verschiedene Werkstoffe, aus welchen Bekleidungsstücke usw. hergestellt werden.

Aus der DE-B Nr. 1173615 sind Wundverbände bekannt, die auf ihrer der Wunde zugekehrten Fläche Silber- oder Aluminiumfolien tragen oder einseitig metallisiert sind. Weiters wird in dieser Druckschrift vorgeschlagen, dass solche Verbände neben örtlich metallisierten Bereichen saugfähige, d.h. nicht metallisierte Bereiche aufweisen sollen, um dem Zweck des Wundverbandes, die Heilung von Wunden zu fördern, gerecht zu werden. Ähnliches schlägt die FR-A Nr. 1133472 vor.

Der Gedanke, auf den verchiedensten Gegenständen Metallschichten vorzusehen, ist mehrfach bekannt. So sind aus der FR-A Nr. 2268540 und der BE-A Nr. 516756 Hitzeschutzanzüge bekannt, die aussenseitig metallische Schichten tragen. Aus der DE-A Nr. 2209281 ist ein Riemen, der innenseitig mit einem Gewebe bedeckt ist, zu entnehmen. Bei all diesen vorbekannten Gegenständen ist nicht ausgeschlossen, dass der Werkstoff, aus dem der jeweilige Gegenstand besteht, mit der Haut eines Trägers in Berührung kommt, so dass Allergien und/oder Hautreizungen entstehen können.

Die Erfindung stellt sich die Aufgabe, einen Gegenstand der eingangs genannten Gattung so auszugestalten, dass Hautreizungen und/oder Allergien ohne nachteilige Beeinflussung der Trageeigenschaften ausgeschlossen sind.

Die Erfindung löst diese Aufgabe dadurch, dass die Schichte eine den Übertritt von Allergenen und/oder Irritantien aus dem Werkstoff auf bzw. in die unverletzte Haut des Trägers verhindernde durchgehende Schichte ist, und dass der Gegenstand entweder ein Uhrband aus Leder oder lederähnlichem Werkstoff, das die Schichte auf dessen Innenfläche, z.B. auf dem Futter des Uhrbandes trägt, ein Bekleidungsstück, eine orthopädische Ware oder eine Prothese ist, das bzw. welche die Schichte zumindest auf seiner bzw. ihrer der unverletzten Haut des Trägers zugekehrten Seite aufweist.

Durch die erfindungsgemäss vorgesehene durchgehende Schichte ist vermieden, dass der Werkstoff, aus dem der Gegenstand gefertigt ist, unmittelbar auf der unverletzten Haut des Trägers aufliegt. Die Schichte verstärkt die Funktion der in bestimmten Bereichen des menschlichen Körpers, z.B. am Armgelenk, wo Armbanduhren getragen werden, oder an der Stirn bzw. der Hüfte, werminderd ausgebildeten Hornhaut, die an sich den Eintritt von Allergenen und/oder Irritantien in tiefere Schichten der Haut verhindert. Darüber hinaus ergibt sich durch die Metallschichte ein Schutz des Gegenstandes vor Feuchtigkeit, z.B. beim Tragen von Lederriemen (Uhrbändern) beim Baden oder wenn der Träger – bei der Ausübung von Sport – schwitzt. Da Schweiss Allergene aus Werkstoffen herauslösen kann und oft erst dadurch die Sensibilisierung ausgelöst wird, kommt dieser Funktion besondere Bedeutung zu. Weiters können durch die Schichte die mechanischen Eigenschaften des Gegenstandes, so z.B. die Abriebfestigkeit, verbessert werden.

Im Rahmen der Erfindung kann vorgesehen sein, dass zwischen dem Werkstoff und der Schichte ein Haftvermittler vorgesehen ist. Als Haftvermittler kann eine Kunstharzschichte, z.B. eine eingebrannte Lackschichte, vorgesehen sein. Durch diese Zwischenschichte werden im Werkstoff allenfalls vorhandene Poren oder Unebenheiten weitgehend geschlossen bzw. ausgeglichen, so dass eine praktische lückenlose Beschichtung möglich ist. Es besteht auch die Möglichkeit, durch Einfärben der Haftvermittlerschichte in einer zur Schichte kontrastierenden Farbe ein Signal für die Zerstörung oder Abnützung der Sperrschicht für die Allergene und/oder Irritantien zu geben.

Normalerweise wird die Metallschichte eine Dicke bzw. die Metallpartikel eine Grösse von 150 bis 500 µm besitzen. Bevorzugte Werte liegen zwischen 200 und 350 µm. Bei einer Aluminiumbeschichtung hat sich z.B. eine Dicke von 260 µm als geeignet erwiesen.

Die Schichte kann auch uneben ausgebildet sein. Hiezu können z.B. kalottenförmige Vorsprünge vorgesehen sein, so dass die Schichte nicht vollflächig anliegt. Die Vorsprünge können in einfacher Weise entweder durch verdickte Bereiche der Schichte oder durch bereichsweises Verdichten derselben gebildet werden.

Gegenstände, wie sie im vorliegenden Fall gemeint sind, können die verschiedensten Sachen sein. Beispiele sind Bekleidungen und Bekleidungsstücke jeder Art, z.B. Arbeits(schutz)bekleidung, Kleidung, die von Ärzten und/oder Schwestern getragen wird, Schutzhandschuhe, Operationshandschuhe, orthopädische Waren, wie über längere Zeit oder ständig zu tragende Stützverbände, Prothese usw. Ein wichtiges Anwendungsgebiet der Erfindung sind Uhrbänder aus echtem Leder oder solche aus lederähnlichen Werkstoffen.

## Patentansprüche

1. Gegenstand aus insbesondere flexiblem Werkstoff, der bei seiner bestimmungsgemässen Verwendung auf der Haut oder der Schleimhaut eines Trägers unmittelbar aufliegt und zumindest in seinem bei bestimmungsgemässer Verwendung auf der Haut oder der Schleimhaut des Trägers aufliegenden Bereich eine Schichte trägt, die aus Metall, insbesondere Aluminium oder einem Edelmetall, vorzugsweise Silber, oder aus Metallpartikeln, insbesondere Aluminium oder einem Edelmetal, vorzugsweise Silber, besteht, die in ein Bindemittel eingebettet sind, dadurch gekennzeichnet, dass die Schichte eine den Übertritt von Allergenen und/oder Irritantien aus dem Werkstoff auf bzw. in die unverletzte Haut des Trägers verhindernde durchgehende Schichte ist, und dass der Gegenstand entweder ein Uhrband aus Leder oder lederähnlichem Werkstoff, das die Schichte auf dessen Innenfläche, z.B. auf dem Futter des Uhrbandes trägt, ein Bekleidungsstück, eine orthopädische Ware oder eine Prothese ist, das bzw. welche die Schichte zumindest auf seiner bzw. ihrer der unverletzten Haut des Trägers zugekehrten Seite aufweist.

2. Gegenstand nach Anspruch 1, dadurch gekennzeichnet, dass zwischen dem Werkstoff und der Schichte ein Haftvermittler vorgesehen ist.

3. Gegenstand nach einem der beiden Ansprüche 1 oder 2, dadurch gekennzeichnet, dass als Haftvermittler bzw. als Bindemittel ein Kunstharz, z.B. ein vorzugsweise eingebrannter Lack, vorgesehen ist.

4. Gegenstand nach einem der Ansprüche 1 bis 3, dadurch gekennzeichnet, dass die Metallschichte eine Dicke bzw. die Metallpartikel eine Grösse von 150 bis 500 µm, insbesondere 200 bis 350 µm, besitzt bzw. besitzen.

5. Gegenstand nach einem der Ansprüche 2 bis 4, dadurch gekennzeichnet, dass der Haftvermittler in einer zur Farbe der Schichte kontrastierenden Farbe eingefärbt ist.

6. Gegenstand nach einem der Ansprüche 1 bis 5, dadurch gekennzeichnet, dass die Schichte z.B. abgerundete Vorsprünge aufweist, die vorzugsweise durch bereichsweises Verdichten der Schichte gebildet sind.

## Revendications

1. Objet, en particulier en matériau flexible, qui, lors de son utilisation conformément à sa destination, repose directement sur la peau ou la muqueuse d'un porteur et qui, au moins dans sa zone reposant, lors de son utilisation conformément à sa destination, sur la peau ou la muqueuse du porteur, porte une couche qui est constituée de métal, en particulier d'aluminium ou d'un métal noble, de préférence d'argent, ou de particules métalliques, en particulier d'aluminium ou d'un métal noble, de préférence d'argent, qui sont noyées dans un liant, caractérisé en ce que la couche est une couche continue qui empêche le passage des allergènes et/ou des irritants à partir du matériau sur et dans la peau intacte du porteur, et en ce que l'objet est un bracelet de montre en cuir, ou en un matériau analogue à du cuir, qui porte la couche sur sa face interne, par exemple sur la doublure du bracelet de montre, ou une pièce de vêtement, un article orthopédique ou une prothèse qui présente la couche au moins sur son côté orienté vers la peau intacte du porteur.

2. Objet suivant la revendication 1, caractérisé en ce qu'un agent adhésif est prévu entre le matériau et la couche.

3. Objet suivant l'une des revendications 1 ou 2, caractérisé en ce qu'une résine synthétique, par exemple un vernis de préférence cuit au four, est prévue comme agent adhésif ou, respectivement, comme agent liant.

4. Objet suivant l'une des revendications 1 à 3, caractérisé en ce que la couche métallique, ou respectivement les particules métalliques, présente, ou respectivement présentent, une épaisseur, ou respectivement une dimension, de 150 à 500 µm, en particulier de 200 à 350 µm.

5. Objet suivant l'une des revendications 2 à 4, caractérisé en ce que l'agent adhésif est teinté en une couleur qui contraste avec la couleur de la couche.

6. Objet suivant l'une des revendications 1 à 5, caractérisé en ce que la couche présente des saillies, par exemple arrondies, qui sont formées de préférence par compression zonale de la couche.

## Claims

1. Article, in particular of flexible material, which during its designated use directly contacts the skin or the mucous membrane of the wearer and which at least in its area contracting the skin or the mucous membrane of the wearer during its designated use is provided with a layer which consists of metal, in particular aluminium or a noble metal, preferably silver, or which consists of metal particles, in particular aluminium or a noble metal, preferably silver, which are embedded in a binder characterized in that the layer is a continous layer capable of preventing the transfer of allergens and/or irritants from the material onto and into the unharmed skin of the wearer respectively and that the article is either a watch strap of leather or leather-like material, which has the layer on its inner surface e.g. the lining of the watch strap, or an article of clothing, an orthopaedic article or a prothesis, which is provided with the layer at least on its surface facing the unharmed skin of the wearer.

2. Article according to claim 1, characterized in that a bonding layer is provided between the material and the layer.

3. Article according to claim 1 or 2, characterized in that a synthetic resin, e.g. preferably a baked enamel, is the bonding layer and the binder respectively.

4. Article according to one of claims 1 to 3, characterized in that the metal layer has a thickness and the metal particles respectively have a size of 150 to 500 µm, in particular 200 to 350 µm.

5. Article according to one of claims 2 to 4, characterized in that the bonding layer is coloured in a colour contrasting to the colour of the layer.

6. Article according to one of claims 1 to 5, characterized in that the layer is provided with for example rounded protruding portions, which preferably are made by locally compressing the layer.